# EUROPEAN PATENT APPLICATION

(11) **EP 1 568 273 A1**
(43) Date of publication of application: **31.08.2005**
(21) Application number: 03777288.6
(22) Date of filing: 05.12.2003
(51) Int. Cl.: A01K 67/027, C12N 5/16

(54) **METHOD OF CONSTRUCTING NONHUMAN MAMMAL HAVING RNAi PHENOTYPE**

(30) Priority: 06.12.2002 JP 2002354715
(71) Applicant: MITSUBISHI CHEMICAL CORPORATION, Tokyo 108-0014 (JP)
(72) Inventor: KATSUKI, Motoya, Minato-ku, Tokyo 106-0032 (JP); ISHIDA, Mitsuyoshi, c/o GENCOM CORPORATION, Machida-shi, Tokyo 194-8511 (JP)
(74) Representative: Sternagel, Fleischer, Godemeyer & Partner
(86) International application number: PCT/JP2003/015594
(87) International publication number: WO 2004/052093

(57) **Abstract**

It is an object of the present invention to develop a novel method for producing mammals such as mice having an RNAi phenotype, thereby enabling the production of RNAi-expressed progenies by a new mechanism to transfer information to progenies, and to improve a method for introducing dsRNA to improve the efficiency of obtaining mammals such as mice having an RNAi phenotype. The present invention provides a method for producing a non-human mammal with suppressed function of a target gene, which comprises injecting the double-stranded RNA (dsRNA) of the garget gene into the nucleus of a cell.

## Description

### TECHNICAL FIELD

The present invention relates to a method for producing a non-human mammal having an RNAi phenotype by using a double-stranded RNA (dsRNA) of a target gene.

### BACKGROUND ART

To date, in order to clarify the functions of DNA at an individual level, a method comprising producing a gene knockout animal and analyzing the phenotype thereof has been applied. However, such a knockout method involves enormous effort and time, and thus, it is not practical for analysis of a large number of gene functions. Accordingly, it is desired that a method for suppressing gene functions at an individual animal level that is more effective and simple than the conventional knockout method will be developed.

The term "RNAi (RNA interference)" is used to mean a phenomenon whereby after RNA (double stranded RNA: dsRNA) formed by conversion of a part of mRNA encoding a part of a certain gene (referred to as a target gene) into a double stand has been introduced into a cell, the expression of the target gene is suppressed. In 1998, the fact that introduction of dsRNA into a living body exhibits action to suppress the expression of the same gene as the introduced gene was discovered in nematodes (Nature, 391 (6669) 806-811, 1998). Thereafter, such phenomenon has also been found in Eumycetes, plants *Nicotiana tabaccum* and *Oryza sativa*, planarias, *Trypanosoma brucei* (J. Biol. Chem., 275 (51) 40174-40179, 2000), the fly *Drosophila melanogaster* (Cell, 95 (7) 1017-1026, 1998), and zebra fish as a vertebrate animal species. Thus, it has been considered that RNAi is a phenomenon that is universally observed, regardless of species.

The technical application of RNAi has been established in nematodes as a gene knockout technique. It has been utilized as a principal means for analyzing genome function using the total nucleotide sequence information obtained through a project for determining the total genomic sequence of a nematode (Nature, 408 (6810) 325-330, 2000; Nature, 408 (6810) 331-336, 2000). Also in the analysis of genome function of mammals, it is expected that RNAi may be used in a method for efficiently suppressing gene expression, which is less burdensome than the gene knockout method in time and manpower.

With regard to mammals, the RNAi effect has been reported for the first time in an experiment wherein dsRNA was injected into an early embryo of a mouse (Nat. Cell Biol., 2 (2) 70-75, 2000). However, such an RNAi effect was observed only in the early embryo, and the effect disappeared when the mouse was born. It is considered that the reason why the RNAi effect disappeared is that dsRNA that had been introduced into a fertilized egg (a one-cell embryo) was then diluted depending on the division and growth of the embryo, and that it became impossible to maintain the concentration necessary for RNAi. Another possible factor is that mammals have mechanisms that biologically differ from those of nematodes.

### DISCLOSURE OF THE INVENTION

It is an object of the present invention to solve the aforementioned problems of the prior art methods. Thus, it is an object of the present invention to develop a novel method for producing mammals such as mice having an RNAi phenotype, thereby enabling the production of RNAi-expressed progenies by a new mechanism to transfer information to progenies. In addition, it is another object of the present invention to improve a method for introducing dsRNA to improve the efficiency of obtaining mammals such as mice having an RNAi phenotype.

The present inventors have conducted intensive studies directed towards achieving the aforementioned objects. First, the present inventors attempted to obtain a non-human mammal having an RNAi phenotype by introducing dsRNA into a site other than cytoplasm. The dsRNA of EGFP was introduced into the nucleus of an EGFP mouse fertilized egg, and such a fertilized egg was then cultured until it became a blastocyst. As a result, a blastocyst with reduced fluorescence was observed. The blastocyst with reduced fluorescence was transferred into the uterus of a recipient mouse. 12.5 days after the pregnancy, the mouse was subjected to dissection, and EGFP fluorescence was observed. As a result, individuals (embryos) with reduced fluorescence were observed. On the other hand, the dsRNA of EGFP was introduced into the cytoplasm of a fertilized egg, and such a fertilized egg was then cultured until it became a blastocyst. As a result, a blastocyst with reduced fluorescence was observed in this case also. However, when this blastocyst with reduced fluorescence was transferred into the uterus of a recipient mouse, and the mouse was then dissected 12.5 days after the pregnancy, it was found that the rate of obtaining embryonic individuals was significantly low. When EGFP fluorescence was observed, reductions in such fluorescence were not found. Moreover, EGFP dsRNA was introduced into the nucleus of an EGFP mouse fertilized egg, and the two-cell stage embryo was then transferred into a recipient mouse. As a result, mice with reduced EGFP fluorescence were found among newborn mice. The present invention has been completed based on these findings.

That is to say, the present invention provides a method for producing a non-human mammal with suppressed function of a target gene, which comprises injecting the double-stranded RNA (dsRNA) of the garget gene into the nucleus of a cell.

Preferably, the double-stranded RNA (dsRNA) of a target gene is injected into the nucleus of a fertilized egg.

In another aspect, the present invention provides a non-human mammal with suppressed function of a target gene which is produced by the aforementioned method of the present invention, or a progeny thereof, or a portion thereof.

In another aspect, the present invention provides a fertilized egg into the nucleus of which the double-stranded RNA (dsRNA) of a target gene has been injected, an embryo developed from the aforementioned fertilized egg, a fetus obtained by transplanting the aforementioned embryo into the uterus or oviduct of a corresponding non-human mammal followed by development, or a progeny thereof, or a portion thereof.

### BRIEF DESCRIPTION OF THE DRAWINGS

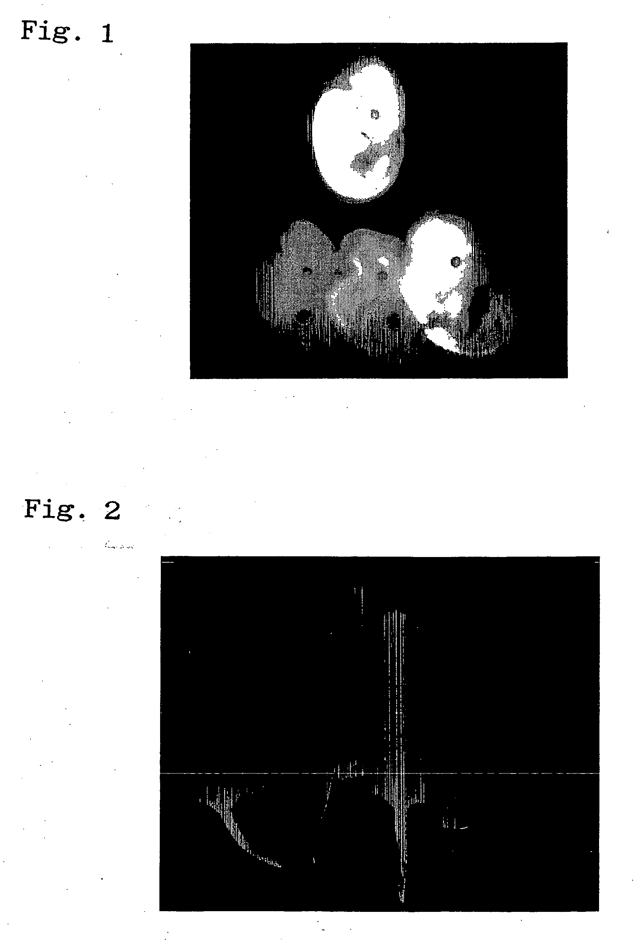

Figure 1 shows the results obtained by obtaining a fetus developed from a fertilized egg into which EGFP dsRNA was injected, and observing the expression of EGFP in the fetus using a fluorescence stereoscopic microscope. The upper case indicates a fetus that did not experience injection. The lower case, left, indicates a fetus with reduced fluorescence; the lower case, center, indicates a fetus with a slight degree of reduced fluorescence; and the lower case, right, indicates a fetus in which fluorescence was not reduced.
Figure 2 shows the results of EGFP fluorescence observation, obtained by applying a 365-nm ultraviolet ray to newborn mice in a darkroom. The two individuals on the left indicate mice that did not experience injection; the two individual in the center indicate mice in which fluorescence was not reduced; the second individual from the right indicates a mouse with reduced fluorescence; and the rightmost individual indicates a mouse with a slight degree of reduced fluorescence.

### BEST MODE FOR CARRYING OUT THE INVENTION

The embodiments of the present invention will be described in detail below.

The present invention relates to a method for producing a non-human mammal with suppressed function of a target gene, which comprises injecting a double-stranded RNA (dsRNA) of the target gene into the nucleus of a cell. The term "with suppressed function of a target gene" is used in this specification to mean that a non-human mammal has an RNAi phenotype.

To date, for the purpose of maintaining the intracellular concentration of introduced dsRNA, the present inventors have constructed a gene by ligating a gene comprising an inverted repeat sequence downstream of a mammalian expression vector and have introduced the constructed gene into a fertilized egg of an EGFP transgenic mouse. Thereafter, they transferred the embryo into the oviduct of a mouse, so as to produce a mouse into which an EGFP dsRNA expression vector gene was introduced. In the thus produced mice, several individuals having a phonotype in which the expression of a target gene (EGFP) was suppressed were observed (Japanese Patent Application No. 2001-46089). However, the efficiency of obtaining such mice was low. Thus, in order to analyze gene functions of an individual mouse using the RNAi effect, further improvements have been desired.

In the present invention, a method for producing a non-human mammal with suppressed function of a target gene (that is, having an RNAi phenotype) by injecting the double-stranded RNA (dsRNA) of the target gene into the nucleus of a cell in a fertilized egg or the like, was adopted. It was found for the first time that a non-human mammal with suppressed function of a target gene can efficiently be produced by applying this method.

### (1) Target gene

Any given gene can be used as a target gene in the present invention. When a non-human mammal with suppressed function of a target gene is produced by the method of the present invention, the target gene is a gene the expression of which is intended to be suppressed. Such target genes include genes that have been cloned but the functions of which are still unknown.

Otherwise, such a target gene may also be a gene of a foreign reporter protein or a gene of a mutant protein thereof. When such a foreign reporter protein gene or its mutant protein gene is used as a target gene, the RNAi effect can easily be detected and evaluated by the method of the present invention for introducing dsRNA into the nucleus of a cell.

Examples of a foreign reporter protein may include an enhanced green fluorescent protein, a green fluorescent protein, aequorin, chloramphenicol acetyltransferase, β -galactosidase, luciferase, and β-glucuronidase.

An example of a mutant protein of such a foreign reporter protein may be a protein that comprises a substitution, deletion, addition, and/or insertion of one or several (for example, 1 to 20, preferably 1 to 10, and more preferably 1 to 5) amino acids with respect to the amino acid sequence of the above-described wild type reporter protein, and which preferably maintains functions equivalent to or greater than those of the wild type reporter protein.

Specific examples of the gene of such a mutant reporter protein used herein may include a gene comprising a deletion of a portion of the nucleotide sequence of a reporter protein gene, a gene comprising a substitution of the nucleotide sequence of a reporter gene with another nucleotide sequence, and a gene comprising an insertion of another nucleotide sequence into a portion of the nucleotide sequence of a reporter gene. The number of nucleotides to be deleted, substituted, or added is not particularly limited. It is generally between 1 and 60, preferably between 1 and 30, and more preferably between 1 and 10. It is desirable that these mutant genes maintain the functions of reporter genes.

A mutant protein gene can be produced by any given methods that have already been known to a person skilled in the art, such as chemical synthesis, genetic engineering, or mutagenesis. Specifically, an agent acting as a mutagene is allowed to come into contact with DNA encoding a natural reporter protein so as to allow the agent to act thereon, or an ultraviolet ray is applied. Otherwise, genetic engineering such as the PCR method is used, thereby obtaining a gene encoding a mutant protein. Site-directed mutagenesis, a genetic engineering method, is particularly effective in that it is a method for introducing a specific mutation into a specific site. Such site-directed mutagenesis can be applied by methods described in Molecular Cloning: A laboratory Manual, 2^{nd} ed., Cold Spring Harbor Laboratory, Cold Spring Harbor, NY., 1989; and Current Protocols in Molecular Biology, Supplements 1 to 38, John Wiley & Sons (1987-1997), etc.

### (2) Double-stranded RNA (dsRNA)

Double-stranded RNA (dsRNA) can be prepared by mixing the sense strand RNA of a target gene with the antisense strand RNA thereof and then annealing both strands. More specifically, first, both template DNA used for transcription of the sense strand RNA of a target gene and template DNA used for transcription of the antisense strand RNA thereof are prepared by gene recombination methods known to persons skilled in the art. Subsequently, transcription of RNA is carried out by common methods using these template DNAs used for RNA transcription. Such transcription of RNA can also be carried out using a commercially available kit such as the RiboMAX Large Scale RNA Production System-T7 (Promega). The reaction product generated as a result of the transcription reaction is subjected to DNase treatment. Thereafter, the transcribed RNA may be purified by common methods such as phenol-chloroform extraction, chloroform extraction, or ethanol precipitation. Finally, the above prepared sense RNA and antisense RNA are mixed preferably in equal amounts, and the obtained mixture is subjected to annealing operations to form a double strand, thereby producing a double-stranded RNA (dsRNA). Excessive single-stranded RNA may be treated with appropriate nuclease (RNase), and dsRNA may be purified by common methods such as phenol-chloroform extraction, chloroform extraction, or ethanol precipitation.

### (3) Introduction of dsRNA into nucleus of cell

The type of a cell into which the dsRNA of a target gene is to be introduced is not particularly limited in the present invention. For the purpose of efficiently producing a non-human mammal, it is preferably a germ line cell. Specific examples of such a germ line cell may include germ cells such as a fertilized egg, an unfertilized egg, sperm, or an initial cell thereof. A more preferred example includes a cell at an early stage of the formation of an embryo in the development of a non-human mammal (more preferably at a stage of a single cell or amphicytula, which is generally before the 8-cell stage). Thus, dsRNA is introduced into the nucleus of such a cell, and an individual is allowed to develop from this cell, so as to produce a non-human mammal with suppressed function of a target gene. In the present invention, dsRNA of a target gene is preferably introduced into the nucleus of a fertilized egg.

Introduction of double-stranded RNA (dsRNA) into the nucleus of a cell is carried out by methods known to persons skilled in the art. For example, such injection into the nucleus can be carried out by microinjection under a phase-contrast microscope.

### (4) Production of non-human mammal

In the present invention, a non-human mammal with suppressed function of a target gene is produced by using a cell into the nucleus of which the dsRNA of the target gene obtained in (3) above has been introduced. The obtained non-human mammal, a progeny thereof, and a portion thereof are also included in the scope of the present invention. In other words, the present invention further relates to an embryo developed from a fertilized egg, an unfertilized egg, sperm, ES cells or the like, into the nucleus of which the dsRNA of a target gene had been introduced, and a fetus obtained by transplanting the aforementioned embryo into the uterus or oviduct of a corresponding non-human mammal followed by development.

Examples of a portion of the above non-human mammal may include cell organella, cells, tissues, and organs of the non-human mammal, as well as the head, finger, hand, foot, abdomen, and tail thereof.

Examples of a non-human mammal may include a mouse, a rat, a hamster, a Guinea pig, a rabbit, a dog, a cat, a horse, a bovine, a sheep, a swine, a goat, and a monkey, but examples are not limited thereto. As such a non-human mammal, rodents such as a mouse, rat, or Guinea pig are preferable, and a mouse and a rat are particularly preferable. Examples of a mouse may include inbred mice such as C57BL/6, DBA2, or BALB/c, and hybrid mice such as B6C3F1 or B6D2F1. ICR is an example of a closed colony. Specific examples of a rat may include Wistar and SD rats.

When double-stranded RNA (dsRNA) is introduced into the fertilized egg of a non-human mammal or a progenitor thereof, the used fertilized egg is obtained by breeding a male non-human mammal with a female non-human mammal of the same type. Such a fertilized egg can be obtained by natural crossbreeding, but it is preferable that the sexual cycle of the female non-human mammal be artificially controlled and that such female then be bred with the male non-human mammal. As a method for artificially controlling the sexual cycle of a female non-human mammal, it is preferable that follicle-stimulating hormone (pregnant mare serum gonadotrophin) be first administered intraperitoneally by injection and that luteinizing hormone (human chorionic gonadotropin) be then administered thereto by injection. The preferred dosage and administration intervals regarding such hormones can be determined as appropriate depending on the type of non-human mammal involved.

After double-stranded RNA (dsRNA) has been introduced into the nucleus of a fertilized egg, the egg is artificially transplanted and implanted into a female non-human mammal. As a result, a non-human mammal into which dsRNA has been introduced is obtained. It is preferred that luteinizing hormone-releasing hormone (LHRH) or an analog thereof is administered to a female non-human mammal, and then the female non-human mammal is bred with a male non-human mammal, so that a fertilized egg into which dsRNA has been introduced can artificially be transplanted and implanted into the pseudopregnant female non-human mammal whose fertilization ability has been induced. The appropriate amount of LHRH or an analog thereof administered, and the period in which a female non-human mammal is bred with a male non-human mammal after the administration of LHRH or an analog thereof, can be selected as appropriate depending on the type of relevant non-human mammals or the like.

The fact that dsRNA exists even in the germ cells of an animal produced after introduction of the dsRNA means that the RNAi effect exists in the progeny of the produced animal. Progeny inheriting the RNAi effect from the above animal is also included in the present invention.

With regard to non-human mammals produced by the method of the present invention, it is expected that the expression of the target gene would be suppressed by the RNAi effect. A model animal wherein functions of a target gene have been knocked out is useful for analysis of the functions of a novel gene and the like.

The present invention will be more specifically described in the following examples. However, the examples are not intended to limit the scope of the present invention.

### EXAMPLES

### Example 1: Preparation of dsRNA

EGFP dsRNA used for injection and HPRT dsRNA used as a control were prepared by the following methods.

### (1) Preparation of template DNA used for transcription of EGFP RNA

pCE EGFP-1 (publication: Takada, T. et al, Selective production of transgenic mice using green fluorescent protein as a marker. Nature Biotech. 15: 458-461, 1997) was cleaved with restriction enzymes *Nco*I and *Dra*I, and separated by 1% agarose gel electrophoresis. Thereafter, an 800-bp band was cut out, and DNA was recovered, so as to use it as a fragment to be inserted. This fragment was ligated to a Litmus 28 vector (New England Biolabs) that had been treated with *Nco*I and *Eco*RV. Thereafter, *Escherichia coli* JM109 was transformed with the above vector, so as to obtain an EGFP LI plasmid into which an EGFP gene had been incorporated. Thereafter, the EGFP LI plasmid was treated with *Spe*I, so as to obtain template DNA used for transcription of EGFP sense RNA. The same plasmid was treated with *Afl*II, so as to obtain template DNA used for transcription of EGFP antisense RNA.

### (2) Preparation of template DNA used for transcription of HPRT RNA

A plasmid pHRT5 containing HPRT cDNA was furnished from ATCC (ATCC Number 37424). A fragment obtained by treating pHRT5 with *Age*I and *Bal*I was treated with *Age*I and *Eco*RV. The thus treated fragment was then ligated to a Litmus 28 vector that had been treated with CIP, so as to obtain an HPRT LI plasmid, into which an HPRT gene had been incorporated. Thereafter, the HPRT LI plasmid was treated with *Spe*I, so as to obtain template DNA used for transcription of HPRT sense RNA. The same plasmid was treated with *Afl*II, so as to obtain template DNA used for transcription of HPRT antisense RNA.

### (3) Preparation of RNA

Transcription of RNA was carried out using the RiboMAX Large Scale RNA Production System-T7 (Promega). The transcription conditions were determined in accordance with the document attached with the kit. Regarding each gene, both sense RNA and antisense RNA were independently transcribed. After completion of the transcription, DNase treatment was carried out under the conditions described in the aforementioned document. Thereafter, the transcribed RNA was purified by phenol-chloroform extraction, chloroform extraction, and ethanol precipitation.

### (4) Preparation of dsRNA by injection

The thus prepared sense RNA and antisense RNA were mixed in equal amounts, and the mixture was then subjected to annealing operations, so as to form a double strand. In order to eliminate a single-stranded RNA that existed in an excessive amount, the resultant product was treated with Mung Bean Nuclease (TaKaRa) and then subjected to phenol-chloroform extraction, chloroform extraction, and ethanol precipitation, so as to purify dsRNA. The purified dsRNA was conserved in a frozen state as 5 µg/µl aqueous dsRNA solution. When used, it is changed into a PBS(-) solution.

### Example 2: Production of fertilized egg

Fertilized eggs were produced by *in vitro* fertilization according to the method of Toyoda et al. (Studies regarding *in vitro* fertilization of mouse eggs, Animal Breeding Magazine 16: 147-151, 1971). That is to say, PMGS and hCG (7.5 units) were injected intraperitoneally to female mice at an interval of 48 hours. 16 to 18 hours after the injection, eggs were collected and then inseminated with the seminal fluid (which was collected approximately 1.5 hours before collection of the egg; approximately 100 to 150 sperm/µl) of an EGFP transgenic mouse (Masaru Okabe et al, FEBS Letters 407 (1997) 313-319). Approximately 6 hours after the insemination, the release of the secondary polar bodies of the eggs and the presence or absence of both male and female pronuclei were confirmed. Thereafter, only fertilized eggs were collected. The obtained fertilized eggs at the pronuclear stage were cryopreserved by a simple vitrification method according to the method of Nakao et al. (1997). The cryopreservation fertilized eggs were melted before undergoing experiments, and they were then subjected to microinjection.

### Example 3: Injection of dsRNA

dsRNA was injected into the pronucleus of the fertilized egg according to the method of Katsuki et al. (Developmental Engineering Experiment Manual, Production Method of Transgenic Mice, 1987). The fertilized egg was transferred into droplets of modified Whitten's medium (mWM). In the case of intranuclear injection, after a male pronucleus had been confirmed under a phase contrast microscope (DMIRB, Leica), approximately 2 pl of the purified dsRNA solution (2.0 µg/µl) was injected into the pronucleus. In the case of intracytoplasmic injection, approximately 2 pl of the dsRNA solution was injected into the cytoplasm. The surviving embryos were transferred into the mWM medium, and the embryos were then cultured under conditions of 5% CO₂, 95% air, and 37°C. Four days after the injection, the expression of EGFP was observed using a fluorescence stereoscopic microscope (MZ. FL III, Leica). As a result, the presence of embryos with a suppressed EGFP expression (embryos with reduced fluorescence) was confirmed. Embryos including such embryos with reduced fluorescence were transferred into the uterus of a pseudopregnant female ICR mouse and then were implanted.

Fourteen days after the injection, the aforementioned pseudopregnant female ICR mouse was subjected to dissection, so as to obtain fetuses. Thereafter, the expression of EGFP was observed in the fetuses using a fluorescence stereoscopic microscope. As a result, it was confirmed that there were several fetuses with a suppressed EGFP expression among the fetuses produced from the fertilized eggs into which the EGFP dsRNA had been injected (Figure 1).

The results obtained by observation of the blastocyst into which EGFP dsRNA had been injected are shown in the following Table 1. The results of embryo transplantation (anatomic observation) are shown in the following Table 2.

**Table 1:**

| Injection of EGFP dsRNA (observation of blastocyst) | | | | | | |
|---|---|---|---|---|---|---|
| dsRNA | Number of Injections | Number surviving | Survival rate | Blastocyst | Incidence rate of blastocyst (%) | Blastocyst with reduced fluorescence |
| Untreated | - | 116 | - | 46 | 39.7 | 0 |
| EGFP (nucleus) | 232 | 161 | 69.4 | 73 | 45.3 | 31 |
| EGFP (cytoplasm) | 321 | 136 | 42.4 | 48 | 35.3 | 37 |

**Table 2:**

| Embryo transplantation (E12.5 anatomic observation) | | | |
|---|---|---|---|
| | Number of transplanted eggs | Number of fetuses recovered | Number of fetuses with reduced fluorescence |
| Untreated | 46 | 8 | 0 |
| EGFP (nucleus) | 51 | 20 | +2, ±2 |
| EGFP (cytoplasm) | 40 | 1 | 0 |

### Example 4: Obtainment of baby mice after dsRNA injection

EGFP dsRNA was injected into the pronucleus and cytoplasm of a fertilized egg by the same method as described in Example 3. The surviving embryos were transferred into the mWM medium, and the embryos were then cultured under conditions of 5% CO₂, 95% air, and 37°C. On the following day, the embryos at a 2-cell stage were transplanted into the oviduct of a pseudopregnant female ICR mouse and then were implanted. In a darkroom, a 365-nm ultraviolet lamp (UVL-56 type, UVP) was applied to baby mice that were born 18 days after the transplantation, and observation of EGFP fluorescence was carried out. As a result, it was found that there were several baby mice exhibiting reduced EGFP fluorescence on their body surfaces among the baby mice into the nuclei of which EGFP dsRNA had been injected (Figure 2).

### INDUSTRIAL APPLICABILITY

The method of the present invention enables more efficient production of a non-human mammal having an RNAi phenotype than conventional methods have allowed. Moreover, in the analysis of genes associated with diseases or the analysis of genes that are targeted for medicaments using the RNAi effect, it becomes possible to suppress genes more reliably than with the use of conventional mice. Thus, it is considered that the method of the present invention greatly contributes to the industry.

## Claims

1. A method for producing a non-human mammal with suppressed function of a target gene, which comprises injecting the double-stranded RNA (dsRNA) of the garget gene into the nucleus of a cell.

2. The method for producing a non-human mammal according to claim 1 wherein the double-stranded RNA (dsRNA) of a target gene is injected into the nucleus of a fertilized egg.

3. A non-human mammal with suppressed function of a target gene which is produced by the method of claim 1 or 2, or a progeny thereof, or a portion thereof.

4. A fertilized egg into the nucleus of which the double-stranded RNA (dsRNA) of a target gene has been injected,

5. An embryo developed from the fertilized egg of claim 4.

6. A fetus obtained by transplanting the embryo of claim 5 into the uterus or oviduct of a corresponding non-human mammal followed by development, or a progeny thereof, or a portion thereof.
